# EUROPEAN PATENT APPLICATION

(11) **EP 4 000 729 A1**
(43) Date of publication of application: **25.05.2022**
(21) Application number: 20208779.7
(22) Date of filing: 19.11.2020
(51) Int. Cl.: B01L 3/00, C12Q 1/6888, G01N 33/569, C12Q 1/6806, C12N 15/10, C12Q 1/6804

(54) **METHOD FOR DETERMINING THE PRESENCE OF A PATHOGEN**

(71) Applicant: TTP PLC, Royston, Hertfordshire SG8 6EE (GB)
(72) Inventor: Crossley, Peter, Royston, Hertfordshire SG8 6EE (GB); Sanders, Giles, Royston, hertfordshire SG8 6EE (GB); Casey, Jonathan, Royston, hertfordshire SG8 6EE (GB); Yata, Keiko, Royston, hertfordshire SG8 6EE (GB); North, Oliver, Royston, Hertfordshire SG8 6EE (GB); Stein, James, Royston, Hertfordshire SG8 6EE (GB)
(74) Representative: Roberts, Philippa Grace

(57) **Abstract**

The invention relates to a pathogen testing system that can analyses multiple samples at once in order to provide guidance on the presence of an infection in a given environment. This reduces the number of individual tests that are needed and provides enhanced confidence as to the levels of pathogen in a given environment.

## Description

### Field of Invention

The invention relates to an apparatus for performing various assays. In particular, it relates to a cartridge based system adapted to assay multiple samples at once with respect to a range of different pathogens.

### Background

The outbreak of the 2020 coronavirus pandemic has highlighted the need for rapid testing of individuals so that the spread of infections can be monitored and, where possible, infected individuals (and those who have had contact with said individuals) can be isolated quickly.

The science behind testing for specific strains of pathogens is well documented. Moreover, there are many examples of test kits adapted to provide an individual with a positive or negative response. However, the testing of large numbers of individuals requires a correspondingly large number of test kits to be produced and distributed. Many test kits are constructed as self-contained reactors to which samples are added, before being sealed and then manipulated so as to bring the sample material into contact with the relevant chemical and biological agents. This avoids exposing users or technicians to the sample or reaction materials.

As a consequence, every test kit requires the necessary quotient of reagents and each kit must be constructed for each sample that is generated. This means that, in order to test large populations, an extremely large number of individual test kits must be manufactured. This places significant burdens on manufacturing equipment, raw materials and distribution channels.

What is required is a method of testing large populations, using fewer test kits, but which still provides information enabling useful decisions to be taken regarding prudent population isolation measures. It is also desirable to provide a method which reliably discounts the need to test large groups of individuals, so that individual test kits can be deployed most effectively (and reduce the total number of test kits required). The invention is intended to overcome, or at least ameliorate, this problem.

### Summary of Invention

There is provided, in a first aspect of the invention, a method of determining the presence of a pathogen within an environment, the method comprising the steps of: i) collating a plurality of first samples from the environment; ii) homogenising the plurality of first samples to form a second sample; iii) reacting a portion of the second sample with an extraction agent to form a third sample; iv) subjecting the third sample to one or more pathogen assay; and v) based on the one or more pathogen assay, determining the presence of a pathogen within the environment.

The inventors have found that, by using multiple samples at once from a particular environment, one can perform a robust pathogen assay and, based on the outcome of one test, make decisions regarding the isolation requirements of multiple individuals with reference to their interaction with said environment. This reduces the number of test kits needed and, as a result of the comparatively large initial sample size, enhances the reliability of results with respect to a given environment.

The term "environment" used herein is intended to refer to a physical space where potential pathogen hosts gather. Typical examples include, but are not limited to: schools, offices, public transportation systems, zoos, aircraft, ships, factories and the like. In particular, this term does not encompass biological spaces, such as the lungs or mouth.

The first samples described herein are typically obtained from potential pathogen hosts. Typically these are humans, but other animals are also envisaged as potential pathogen hosts. Typically, the method does not include the step of taking the sample directly from the pathogen host. Samples may also be taken from the environment itself rather than directly from potential pathogen hosts. As one skilled in the art would appreciate, pathogens can survive for prolonged periods of time on surfaces and in the atmosphere. It may be the case that samples obtained directly from potential pathogen hosts, and samples obtained from the environment itself (i.e. indirectly taken from a pathogen host), are used together.

The term, "homogenising" as used herein refers to the combination of the biological material from each of the first samples. This combination does not necessarily mean that the second sample possess a uniform distribution of ingredients throughout. It is simply intended that the second sample contains all the biological content from each of the first samples so that, all of this biological material can be exposed to a single instance of the necessary treatment and pathogen assay steps. Typically, the second sample has a uniform distribution the contents of all of the first samples.

In step iii), a portion of the homogenised second sample is reacted with an extraction agent in order to isolate any relevant biological markers for subsequent analysis by the pathogen assay. Because the second sample is a homogenised mixture of all of the first samples, the extraction agent is exposed to a broad spectrum of biological material (that from all of the first samples) in a single reaction. Any material successfully extracted by the extraction agent is typically then amplified before being delivered to the pathogen assay. The portion of the homogenised second sample that is used in step iii) is typically greater than 30 vol.%, more typically greater than 50 vol.% and in some instances greater than 70 vol.%. Often, this portion will be in the range of 75% vol. to 100 vol.%, and more typically in the range of 80 vol.% to 90 vol.%. Often, "substantially all" of the second sample will be used in step iii). Whilst not essential to use the total volume of the second sample, as explained above, many of the benefits of the invention arise from using the combined total of first samples as a source of biological material for testing. Typically, "substantially all" means in the range of 95 vol.% to 100 vol.%, and most typically 98 vol.% to 99.9 vol.%.

For the avoidance of doubt, the term "vol.%" as used herein with respect to the second sample refers to the volume percentage of liquid and solubilised biological matter present following homogenisation. As one skilled in the art would appreciate, the first samples are often provided on a suitable carrier, such as a swab or cotton pad. Said carriers are not relevant to the downstream analytical process but can be placed in the sample receiving chamber. Hence, the total volume of the homogenised second sample is typically equal to the reagent used to solubilise the first samples (added to the sample receiving chamber) plus any residual liquid present within the carriers i.e. it excludes the carriers themselves.

Typically, the homogenisation step solubilises the plurality of first samples so as to extract as much of the biological materials therefrom, and create a solution of said biological material as the second sample. Various techniques may be employed to encourage the solubilisation of biological material from the first samples. For example, the sample receiving chamber may be equipped with a stirrer to mix the samples together with one or more reagents. Alternatively, the reagents may be washed back and forth over the first samples, for instance, between a reagent storage chamber and the sample receiving chamber.

Depending upon the type of first samples used, it is possible that the homogenisation step also includes a maceration step. However, this is typically only in situations where solid carriers for the first samples are not employed (as fragmentation of such materials during maceration can result in large particles that are clog filters or enter the fluid manifold. Such particles can occlude, or block, conduits within the fluid manifold reducing, or preventing, proper functioning of the cartridge.

The term "extraction agent" as used herein, is intended to refer to a substance which sequesters certain biological or chemical components, or categories of components, which are indicative of a particular pathogen to be detected. As one skilled in the art would appreciate, different pathogens can be detected with reference to a range of different tell-tale biomarkers or combination of biomarkers. A typical example of an extraction agent would be, for example, an immobilised antibody or other such co-operative agent that binds the target biomarkers. The specificity of the extraction agent need not be unduly targeted. The extraction agent may, for instance, capture all genetic information from the portion of the second sample or a fraction of the genetic material (e.g. RNA).

It is often the case that step iii) also comprises a further step wherein the components extracted by the extraction agent are amplified, i.e. to increase their concentration. This increases the ability of the pathogen assay to detect the relevant biomarkers and hence increases the accuracy of the pathogen assay.

Reference to a "pathogen assay" as described herein is intended to cover the analytical tool needed to detect particular markers in treated sample, i.e. those markers indicative of the presence of a particular pathogen species. Whilst multiple assays may be combined on a given cartridge, it is often the case that the cartridge comprises one pathogen assay.

As one skilled in the art would appreciate, it is often necessary to condition samples to ensure that the key biological material (or relevant markers) is available to interact with the extraction reagents and assaying equipment. For example, the method may include a cell lysis step in order to liberate the contents of cells. Usually, a cell lysis step may take place before step iii).

There is no particular restriction as to the kind of pathogen assay that is employed in the invention. One skilled in the art would appreciate that a variety of different tests can be performed in order to determine whether a given pathogen, or pathogens, are present within a sample. Indeed, the invention is not limited to the presence of a single pathogen assay. Multiple assays may be included, for instance to detect multiple strains of the same pathogen or to detect different pathogens. That said, the pathogen assay is typically chosen to detect a virus, a bacteria, a fungus or a parasite; most typically the pathogen will be a virus. Most commonly, the pathogen is a human pathogen. Further, the pathogen is typically a highly infectious pathogen, such as foot and mouth disease or influenza. It is typically the case that pathogen assays used in the invention are selected from: nucleic acid detection (such as RNA or DNA detection), direct or indirect antibody staining, antigen detection, protein detection, a lateral flow assay, an optical assay (especially preferred where the pathogen is a bacteria), an enzyme-linked immunosorbent assay (ELISA) or a combination thereof. Of these, nucleic acid detection is typically employed. Examples of pathogen assays compatible with the invention include, but are not limited to: RT-PCR, Endpoint PCR, Isothermal amplification (e.g. LAMP), Protein assay, Optical monitoring of pathogenic particles (e.g. DLS) where nucleic acid amplification is employed as the whole or part of the assay. Detection may be real-time or endpoint and the detection methodology may include: fluorescence, pH change measurement, electrochemical assay of labelled nucleotides, or combinations thereof.

Once step iii) has been completed, the third sample is eluted so as to disassociate the biomarkers or category of biomarkers from the extraction agent and deliver said third sample to the pathogen assay for analysis. The pathogen assay detects the relevant biological material from the third sample, if indeed they are present. For instance, in the case where the pathogen assay employs a nucleic acid detection system, the entire genetic material from the homogenised second sample may have been extracted to create a third sample; and then, subjected to the pathogen assay for analysis. This process of extracting all relevant material, and utilising the sum total of all the biological material in the first sample in the pathogen assay, is advantageous over many prior art systems because more of the original material is preserved. Moreover, this approach is counterintuitive because it does not allow the user to identify which one or more of the first samples, specifically, was the cause of a positive indication. However, this is not entirely necessary to inform appropriate isolation decisions. Moreover, the enhanced accuracy of pathogen detection achieved with this approach, means that the invention can serve as an excellent preliminary testing system for a given environment. Therefore, used in tandem with individual test kits, the invention can enhance pathogen detection and reduce the overall number of kits necessary to protect a population.

The indication provided by the claimed method is not particularly limited, so long as it provides information regarding the presence of one or more relevant pathogens in the environment. Typically, the indication will have a binary output with respect to each of the one or more pathogens for which the pathogen assay is configured. Often, the indication relates to the presence of one particular pathogen.

It is often the case that, as part of the method of the invention, the process involves a step of amplifying the biological material (or at least a relevant portion of the material or markers indicative of a particular pathogen) extracted from the second sample. Typically, this amplification process relates to genetic material and usually involves a polymerase chain reaction (PCR) or suitable isothermal method (such as, LAMP). The person skilled in the art would be familiar with the techniques needed to perform such processes and the associated reagents that would be required. Alternatively, a protein assay or ELIZA may be used.

It is often the case that the pathogen for which the pathogen assay is testing is a coronavirus. More typically, it is SARS-Cov 2 (i.e. that which is responsible for COVID-19).

In some situations, the first samples have been obtained from two or more biological hosts from within the environment. It is possible that more than one of the first samples have been obtained from the same potential pathogen host. For instance, a range of different biological materials may be taken from the same potential pathogen host (e.g. saliva, blood, faeces). However, it is more commonly the case that the first samples comprise biological material obtained from at least two different individuals from the same environment. This enhances the accuracy of the assessment of the status of the environment, i.e. with respect to the presence of a pathogen, will be determined. Moreover, it is usually the case that each of the first samples has been obtained from a different source within the environment. The term "source" is intended to refer to unique locations within an environment where a pathogen may be present; and, each potential pathogen host in said environment. More typically, it is the case that each of the first samples has been obtained from a different potential pathogen host. This ensures that the second sample that is ultimately generated is most representative of the potential pathogen hosts in a given environment and so increases the accuracy of the output.

There is no particular limitation on the number of first samples that can be included within the sample receiving chamber. However, typically, the number of first samples is in the range of 2 to 100, more usually 3 to 60, more typically 5 to 50, even more typically 10 to 45, and most typically 20 to 40. This has been found to strike an effective balance between, on the one hand, obtaining an optimally representative sample and, on the other hand, the need to obtain results efficiently.

It is often the case that biological material obtained from the potential pathogen host in the first samples is selected from: saliva, blood, mucus, urine, faeces or combinations thereof. Of these, it is usually the case that the biological material is saliva, blood or mucus; more typically saliva or mucus; and most typically saliva. The biological material may be carried upon a suitable carrier, e.g. a swab, a cotton pad or universal transfer fluids. Typical regions where swabs may have been applied to collect biological material include nasopharyngeal, lower nose, upper nose and locations in the mouth. Carriers can be loading into the cartridge of the invention, there is no need to perform a separate extraction process before delivery to the cartridge.

In an alternative embodiment of the invention, the sample receiving chamber can be provided with a pre-homogenised sample representative of biological material from a plurality of potential pathogen hosts. For instance, sewage from a given environmental or swimming pool water may be sampled using the invention.

There is provided, in a second aspect of the invention, an apparatus for performing the method according to the first aspect of the invention, the apparatus comprising: i) a cartridge adapted to receive the first samples; and ii) means for actuating the cartridge; wherein the cartridge comprises: a) a sample receiving chamber adapted to receive a plurality of first samples; b) one or more reagent storage chambers; c) a fluid manifold adapted to receive fluid from the sample receiving chamber and the one or more reagent chambers; d) a pathogen assay in communication with the fluid manifold; and e) a means for indicating an output of the pathogen assay.

The term "cartridge" as used herein is intended to take its usual meaning in the art. That is to say, an element into which the first samples can be introduced and within which the operations necessary for the pathogen assay can be performed. Typically, the cartridge comprises a hard outer casing which allows the cartridge to interface with the actuation means to operate the cartridge. The cartridge is typically sealable such that, once the first samples have been introduced into the sample receiving chamber, the cartridge can be hermetically sealed. This is advantageous as it creates a fixed volume within the cartridge, permitting the contents to be moved around manifold of the cartridge in a controlled fashion.

Often, the cartridge will be configured for single use. For instance, the cartridge may have one or more frangible portions which, on engagement with the means for actuating the cartridge (or completion of the viral assay), is broken to prevent repeated use of a contaminated cartridge.

Often the cartridge has a rigid construction and is constructed so as to have a single configuration in which the cartridge successfully interfaces with the actuation means. This minimises the likelihood of human error when operating the apparatus. Moreover, it is often the case that the cartridge is configured such that, in use, the orientation of the one or more reagent storage chambers eject their contents in a downward direction. This is advantageous as it ensures that any residual air present within the reagent storage chambers is not introduced into the fluid manifold, which might stifle the process. Moreover, air trapped in the fluid manifold can escape into the reagent storage chambers. As explained with respect to the first aspect of the invention, it is typically the case that the cartridge comprises a series of reagents. This avoids contamination with an external environment between manufacture of the cartridge and its point of use. As such, it is typically the case that the cartridge further comprises the one or more reagents. However, in some embodiments of the invention, one or more of the reagent may be delivered to the cartridge, for examples from the actuation means. In some embodiments, the reagents may be provided in the cartridge (e.g. as a solid or concentrate) but a suitable solvent may be provided to the cartridge, e.g. by way of the actuation means, in a hermetically clean fashion. For instance, solvent may be injected into one or more of the reagent storage chambers, for instance via a sealable membrane, prior to actuation of the cartridge. That said, in most embodiments, all reagents are provided in the cartridge. Moreover, the reagents are typically liquids, more often solutions of reagents.

The means for actuating the cartridge are often provided in the form of an instrument comprising a series of actuators. The actuators are able to engage with the relevant portion of the cartridge when the instrument and cartridge are connected. Typically, the instrument comprises a cavity adapted to receive the cartridge in a preferred orientation. The actuators engage with the cartridge so as to manipulate the contents of the cartridge and control the conditions within the cartridge. For instance, magnets may be employed to manipulate magnetic components within the fluid manifold. Actuators often physically engage with the various reagent chambers to move fluid around the cartridge. For instance, in a typical embodiment, the actuators communicate with the plungers present within the reagent storage chambers. The instrument may also include an actuator in the form of a heat element adapted to heat portions of the cartridge in use.

In addition, the instrument is typically equipped with a user interface. This not only displays information regarding the progress of the assay, but also conveys readouts to the user. The instrument is also typically programmable so that actuator's manipulation of the cartridge can be fine-tuned to suit a given scenario. Moreover, the instrument may be compatible with several different cartridge designs, with different biochemistries. Accordingly, the programmability of the instrument allows it to control the operation of different cartridges possessing different biochemistries as needed. As one skilled in the art would appreciate, the instrument is also typically equipped with suitable sensors to monitor conditions within the cartridge.

As explained previously, a person skilled in the art would be aware of the pathogen assays that would be compatible with the invention. Similarly, the reagents that are typically employed in such assays would also be familiar to the skilled person. Accordingly, reagents that may be stored on the cartridge include, but are not limited to: lysis buffers, binding buffers, magnetic beads, washing buffers, elution buffers, LAMP reagents, or combinations thereof.

There is provided in a third aspect of the invention, a cartridge for use with the apparatus according to the second aspect of the invention, the cartridge comprising: a) a sample receiving chamber adapted to receive a plurality of first samples; b) one or more reagent storage chambers; c) a fluid manifold adapted to receive fluid from the sample receiving chamber and the one or more reagent storage chambers; d) a pathogen assay in communication with the fluid manifold; and e) a means for indicating an output of the pathogen assay.

The sample receiving chamber is adapted to receive a plurality of first samples. Typically, this means that the sample receiving chamber has capacity to store volume of first samples in the range of 20 ml to 200ml, more typically 30 to 100ml. More typically, the first sample is adapted to receive two or more carriers, such as swabs. As one skilled in the art will appreciate, it is common practice to obtain samples from patients and potential pathogen hosts using swabs. Accordingly, the sample receiving chamber is usually configured to accommodate up to around 60 swabs, more typically up to 50 swabs, even more typically up to 40 swabs, often in the range of 30 to 40 swabs. Usually, the sample receiving portion can be hermetically sealed with a lid. The sample receiving portion also often comprises a filter. As one skilled in the art will appreciate, the first samples will often include large particulate matter (e.g. fibres from swabs, dirt and the like). Such matter can occlude or block the fluid manifold and so the filter prevents such matter from hindering performance of the method described herein.

The cartridge comprises a fluid manifold which comprises a number of channels through which fluid can flow. The sample receiving chamber is in fluid communication with the fluid manifold such that, in use, the first samples can be washed and the resulting solution can pass through the fluid manifold, reacting with the various reagents as it goes, until it contacts the pathogen assay having been suitably processed. Typically, each of the one or more reagent storage chambers is in communication with the fluid manifold.

As one skilled in the art would appreciate, there are often multiple reactions that must necessarily take place before a sample can be successfully assayed. Moreover, many of these reactions cannot be performed simultaneously and so must be performed in separate regions of the fluid manifold sequentially. As such, the fluid manifold will typically comprise one or more reaction regions in which one or more treatment process can occur prior to delivery to the pathogen assay. It is often the case that each of said one or more reaction regions are separated by valves. There is no particular limitation on the kind of valves used but it is often the case that the configuration of the valves is controllable by the actuation means. Alternatively, one or more of the valves may be replaced in favour of controlled operation of the one or more reagent storage chambers. The flow of reagents from the reagent storage chambers to the fluid manifold can be controlled using an actuation means. By coordinating the actuation of different reagent storage chambers, one can create relative positive and negative pressure within the fluid manifold that effectively replace the function of the valves. In some embodiments, a combination of these two approaches will be adopted.

The fluid manifold typically comprises at least one mixing region and at least one reaction region. In some embodiments, the mixing region and the at least one reaction region are the same. It may be the case that the fluid manifold comprises one or more magnetic beads. Said magnetic beads can be actuated using an external magnetic field to promote movement of fluid through the fluid manifold. The magnetic beads are usually held within the mixing region. Moreover, as one skilled in the art would appreciate, magnetic species can be functionalised so as to enhance the detection of biological markers indicative of the presence of a pathogen.

Accordingly, in some embodiments, the cartridge comprises reagents within in the reagent storage chambers. Once the necessary species have become bound to the beads, superfluous material can be removed from the sample by washing with suitable reagents. Often, two or more washes will be performed and each wash may be conducted using a different reagents or different concentration of reagents to ensure the all superfluous material is removed from the sample prior to delivery of the third sample to the pathogen assay. The third sample is detached from the beads usually by means of a specific elutant which promotes disassociation from the beads.

It is typically the case that the reagents and sample are thoroughly mixed at each stage of the process. Accordingly, it is desirable for the fluid manifold to have a common mixing region to which reagents can be added sequentially, so as to ensure reactions have proceeded with maximum efficiency whilst avoiding the need for multiple separate mixing apparatus within the manifold. Magnetic beads are particularly useful in this regard, as they can function both as a means of agitating the mixture and retaining key markers prior to delivery to the pathogen assay.

Usually, the cartridge comprises one or more reagent storage chambers, each of which may be manipulated using one or more actuators selected from: mechanical actuators, pneumatic actuators, hydraulic actuators, electrical actuators, or combinations thereof. The role performed by the actuators with regard to the reagent storage chambers is to move reagents from one place to another. This can be to promote mixing within a given reaction region or to move the sample from a first reaction region to a second reaction region within the fluid manifold. It is also the case actuators communicate with the valves separating the reaction regions within the fluid manifold. As such, by controlling both the valves and the reagent movement, the necessary reactions can be performed within the cartridge without exposing the cartridge contents to an external environment. It is often the case that the actuators are mechanical or pneumatic actuators.

It is typically the case that the various reagent storage chambers provided in the cartridge are equipped with a plunger which can be acted upon by the actuators so as to expel (or draw) the contents of a given chamber into another chamber and/or the fluid manifold. Accordingly, the actuators can communicate with the plunger, thereby controlling of how the cartridge performs.

Whilst there is no particular restriction on the choice of indication means that are employed in the cartridge of the invention, most often the indication will take the form of a binary change such as an optical change, most typically a colour change with one colour indicative of a negative result and another colour indicative of a positive result. In order that the results might be observed, the cartridge may comprise a transparent window. Accordingly, visual inspection by a user of the apparatus or a corresponding absorption (or transmittance) of light (and subsequent detection and processing) may communicate the results.

In order to aid understanding, preferred embodiments of the invention will now be described with respect to the following figures and examples.

### Description of Figures

Figure 1 shows a schematic diagram of the cartridge.
Figure 2 shows a schematic diagram of the process performed within the cartridge.
Figure 3 shows an operational schematic of the cartridge with respect to a typical actuation method.

### Detailed Description

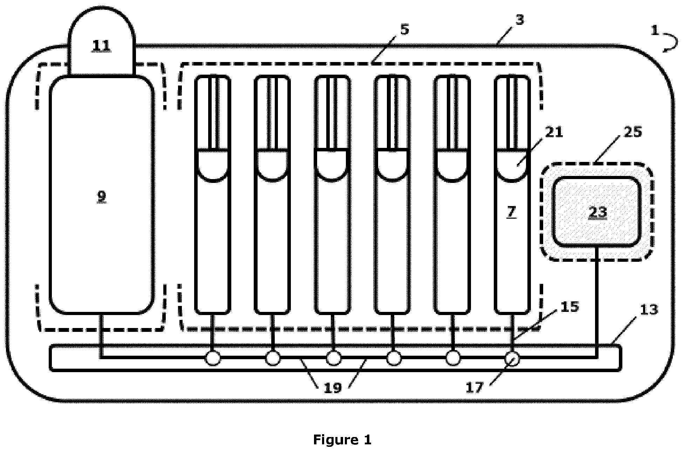

Figure 1 shows an exemplary cartridge **1** for use with the invention. The cartridge comprises a plastic casing **3** which includes a series of internal integral mouldings **5** adapted to receive a series of syringes **7,** each containing a reagent (not shown) for use in the performance the relevant pathogen assay. The cartridge comprises a first sample receiving chamber **9** into which first samples (not shown), typically born upon a carrier such as a swap, are placed in use. This chamber is sealed using a screwable lid **11.**

Each of the syringes **7,** interface with a fluid manifold **13** via separate conduits **15** each equipped with a valve **17.** Moreover, each of said syringes **7** communicate immediately with a different reaction region **19** within the fluid manifold **13.** The plungers **21** associated with each of the syringes **7** are adapted to engage with a corresponding portion of the actuation means (not shown) so that, in use, the movement of the plungers controls the flow of reagents and sample through the fluid manifold **13.** The casing **3** of the cartridge **1** is fashioned such that it can only be correctly inserted in one orientation.

Also shown in Figure 1 is an assay **23** to which reagents and treated sample can be delivered. The assay is positioned behind a transparent window **25** such that, when a colour change (or other such indication is produced by the assay) as an indication of the presence of a given pathogen, an observer (or the actuation means) can read the results without opening the cartridge **1.**

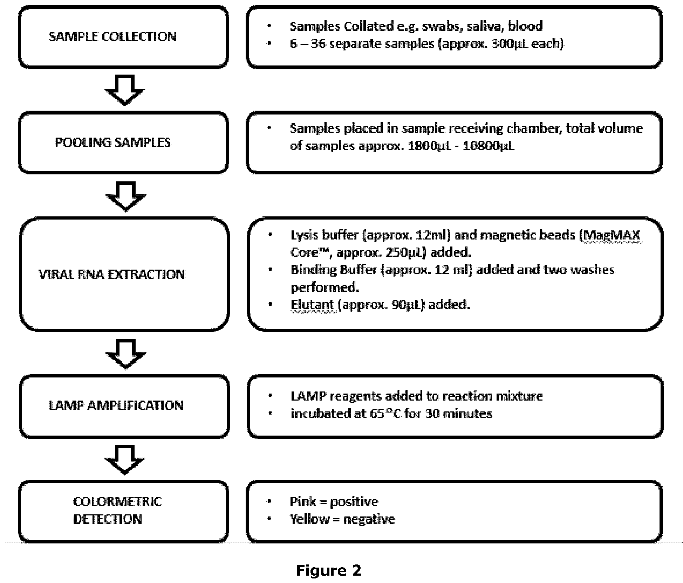

Figure 2 describes a typical process used to analyses samples in accordance with the invention. Initial samples were provided on SalivaBioOral^{™} Swabs (from Salimetrics^{™}). Samples from between 6 and 36 potential pathogen hosts were supplied. A ThermoFischer MagMAX CORE^{™} Nucleic acid purification kit was used to extract viral DNA from the initial pool of samples. Subsequently, a NEB SARS-CoV-2 Rapid Colorimetric LAMP Assay Kit (targeting N and E genes) was employed and the mixture was heated to 65°C for 30 minutes. The results of said test were displayed by means of a colour change.

Regarding the instrument, a cavity adapted to receive the cartridge in a particular orientation (such that air is able to escape from the fluid manifold up into the reagent storage chambers) is provided within which the cartridge can be docked. Surrounding the cavity are a plurality of mechanical actuators which engage with the plungers **21** of the cartridge. The instrument also includes heating elements which, in use, are positioned adjacent to those reaction regions of the fluid manifold where a temperature change is required (e.g. to promote PCR). The instrument is also equipped with a user interface which tracks the progress of the assaying procedure and provides visual feedback to a user regarding the progress. Typically, the user interface also provides an output with respect to the result of the assay. When the cartridge is actuated upon by the instrument, it will lock the cartridge in place to prevent inadvertent removal of the cartridge midway through testing. The instrument also includes a photodetector, which analyses the output from the pathogen assay, and is configured to relay this information to the user interface to display to a user.

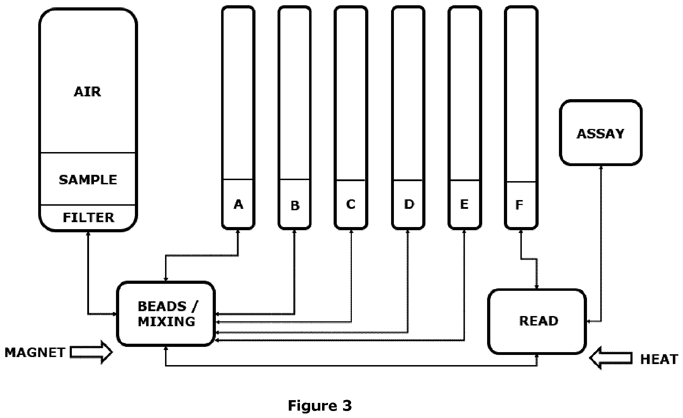

Figure 3 shows a schematic of a possible operating mode for the instrument and associated actuators. Each of solutions A to F are as follows: A (a cell lysis agent); B (a binding agent); C (a first washing); D (a second washing agent, which can be the same as the first but is often different); E (an elutant); F (LAMP reagents for PCR). The actuation operation me consist of the following steps:
1) Hold beads;
2) Push sample plunger and pull lysis plunger simultaneously to mix sample and lysis buffer and to transfer some air to the lysis syringe;
3) Release beads (remove the magnet);
4) Push lysis plunger and pull binding plunger simultaneously to mix sample, beads, lysis and binding buffer. Reverse and repeat actuations until sufficiently mixed;
5) Engage magnet (there may be a wait step at this point);
6) Operate lysis and binding plungers (back and forth) to flow the beads past the magnet and allow their capture. At the end of the last stroke depress the (say) binding actuator sufficiently far to pass air through the chamber whilst leaving some air in the binding syringe;
7) Release the beads (remove the magnet);
8) Push wash 1 plunger and pull binding plunger. Repeat back and forth to process the beads;
9) Engage magnet (there may be a wait step at this point;
10) Operate wash 1 and binding plungers (back and forth) to flow the beads past the magnet and allow their capture. At the end of the last stroke depress the (say) binding actuator and pull the sample plunger sufficiently far to pass all of the waste into the sample syringe and some air through the chamber whilst leaving some air in the binding syringe;
11) Repeat steps (7) to (10) using wash 2 and binding plungers;
12) Release the beads;
13) Push elution plunger and pull binding plunger. Repeat back and forth to elute the beads. Finish this step with the liquid in the elution syringe;
14) Engage magnet (there may be a wait step at this point);
15) Push elution syringe and pull Test syringe to transfer the required volume of Eluate (likely 5ul -10ul) into the Test syringe (may be repeated back and forth to capture beads);
16) Push LAMP plunger and pull test plunger back and forth to pass the liquids though the 'Read' chamber(s) and mix eluate and LAMP reagents. Finish with the read chamber filled;
17) Test is concluded by inducing the conditions required for a LAMP reaction to take place in interrogating with optical components for a readout.

## Claims

1. A method of determining the presence of a pathogen within an environment, the method comprising the steps:
i) collating a plurality of first samples from the environment;
ii) homogenising the plurality of first samples to form a second sample;
iii) reacting a portion of the second sample with an extraction agent to form a third sample;
iv) subjecting the third sample to a pathogen assay; and
v) based on the pathogen assay, determining the presence of a pathogen within the environment.

2. A method according to claim 1, wherein the pathogen is a coronavirus; typically SARS-CoV-2.

3. A method according to claim 1 or 2, wherein the first samples have been obtained from two or more biological hosts from within the environment; and typically wherein each of the first samples has been obtained from a different biological hosts from within the environment.

4. A method according to any of claims 1 to 3, wherein the pathogen assay is a nucleic acid detection assay.

5. An apparatus for performing the method any preceding claim, the apparatus comprising:
i) a cartridge adapted to receive the first samples; and
ii) means for actuating the cartridge;
wherein the cartridge comprises:
a) a sample receiving chamber adapted to receive a plurality of first samples;
b) one or more reagent storage chambers;
c) a fluid manifold adapted to receive fluid from the sample receiving chamber and the one or more reagent chambers;
d) one or more pathogen assays in communication with the fluid manifold; and
e) a means for indicating an output of the pathogen assay.

6. An apparatus according to claim 5, wherein the cartridge further comprises the one or more reagents; and typically wherein the one or more reagents are liquids; most typically wherein the one or more reagents are selected from: lysis buffers, binding buffers, magnetic beads, wash buffers, elution buffers, LAMP reagents, or combinations thereof.

7. A cartridge for use with the apparatus according to claim 5 or claim 6, the cartridge comprising:
a) a sample receiving chamber adapted to receive a plurality of first samples;
b) one or more reagent storage chambers;
c) a fluid manifold adapted to receive fluid from the sample receiving chamber and the one or more reagent chambers;
d) a pathogen assay in communication with the fluid manifold; and
e) a means for indicating an output of the pathogen assay.

8. A cartridge according to claim 7, wherein the fluid manifold comprises one or more reaction regions in which one or more treatment process can occur, typically wherein the one or more regions are separated by valves.

9. A cartridge according to claim 7 or claim 8, wherein the one or more reagent storage chambers further comprise one or more actuators selected from: mechanical actuators, pneumatic actuators, hydraulic actuators, electrical actuators, or combinations thereof; wherein the actuators are adapted to communicate with the means for actuating the cartridge; typically wherein each of the one or more reagent storage chambers comprises a mechanical actuator comprising a plunger.

10. A cartridge according to any of claims 7 to 9, wherein the sample receiving chambers includes one or more mixing means.

11. A cartridge according to any of claims 7 to 10, wherein the means for indicating an output of the pathogen assay is an optical change, such as a colour change or fluorescence.

12. A method of diagnosing one or more individuals with an infection with respect to a given pathogen, using the method of any of claims 1 to 4.

13. A method of diagnosis according to claim 12, where in the infection is COVID-19.
